# EUROPEAN PATENT SPECIFICATION

(11) **EP 2 453 820 B1**
(45) Date of publication and mention of the grant of the patent: **29.06.2016**
(21) Application number: 10800165.2
(22) Date of filing: 15.07.2010
(51) Int. Cl.: A61B 17/80, A61B 17/86, A61B 17/88

(54) **ORTHOPEDIC IMPLANTS SYSTEM INCLUDING A VARIABLE AXIS LOCKING MECHANISM**
ORTHOPÄDISCHES IMPLANTATSYSTEM MIT EINEM VARIABLEN ACHSENSPERRMECHANISMUS
SYSTÈME D'IMPLANTS ORTHOPÉDIQUES COMPRENANT UN MÉCANISME DE BLOCAGE À AXE VARIABLE

(30) Priority: 15.07.2009 US 270980 P; 10.03.2010 US 339857 P; 07.07.2010 US 399113 P; 13.07.2010 US 804062
(43) Date of publication of application: 23.05.2012
(73) Proprietor: Orthohelix Surgical Designs, Inc., Medina, OH 44256 (US)
(72) Inventor: LEWIS, Derek S., Copley Ohio 44321 (US); LEITHER, Andrew J., San Diego California 92122 (US); DILIBERTO, Rebecca F., OH 44121 (US); ALTENAU, Elizabeth, Medina Ohio 44256 (US); HEAVENER, Jackson R., Wadsworth Ohio 44281 (US)
(74) Representative: Grand, Guillaume
(86) International application number: PCT/US2010/001991
(87) International publication number: WO 2011/008286

(56) References cited:
- WO-A1-2006/102110
- WO-A1-2006/103245
- US-A- 4 456 010
- US-A- 5 134 909
- US-A1- 2005 149 040
- US-A1- 2006 106 398
- US-A1- 2006 189 990
- US-A1- 2006 235 400
- US-A1- 2008 114 359
- US-A1- 2008 114 359
- US-A1- 2008 119 895
- US-A1- 2008 119 895
- US-A1- 2009 192 549
- US-B2- 7 052 499

## Description

### FIELD OF THE INVENTION

This present invention relates to an orthopedic implant system having a mechanism for allowing a fastener, such as a screw or a peg, to be used in an orthopedic implant with the long axis of the screw at a variable angle to the orthopedic implant and subsequently to be locked into a desired orientation. The mechanism comprises an assembly, which is used in a threaded hole in the plate that would otherwise provide for a locking relationship with a locking screw at a fixed angle. Thus the assembly provides the option of either fixed angle locked fixation or locked variable angle fixation using the same plate hole. In addition, the system includes a variable angle non-locking screw that can be used in the same threaded plate hole, allowing selectively for three types of fixation in a single location within a plate hole. The system also include a fastener driver not claimed has a tip that will drive a fastener at a variable angle relative to the drive axis of the driver, and further includes a depth limiting feature that will inhibit the fastener or fastener assembly from being driven too deeply relative to the plate hole.

### BACKGROUND OF THE INVENTION

WO 2006/102110 A1 discloses a screw head adapter. The bone plate has a hole with an angle less than about 30 degrees. The system includes a screw with a head and a screw head adapter. The screw head adapter has an inner portion adapted to mate with the head of the screw and an outer portion with a shape corresponding to the angle of the hole. An assembly of the screw and the screw head adapter connect the bone plate to the bone

US 7,052,499 discloses an apparatus for bone fracture fixation. It discloses a fastener with a separate threaded head member. The threaded head member is screwed down on external threads of the fastener after the fastener has been screwed into the bone. US 2008/114359 A1 discloses a polyaxial locking mechanism.

WO 2006/103245 A1 discloses a large-fragment plate comprising at least one fixed, angularly stable threaded borehole used to receive a large-fragment screw.

US 2008/119895 A1 concerns a locking plate intended to be implanted at a bone site and a method for producing and implanting this device. At least one expandable bushing is engaged with a through-hole in the plate. The bushing includes an exterior surface with at least one recess and a passageway including a threaded interior surface.

US 2006/235400 A1 concerns a bone plate having an underside on the side of the bone, an upper side and a plurality of holes in the plate connecting the underside with the upper side, with a central hole axis.

US 2006/116678 A1 discloses a self-locking osteosynthesis device includes a plate equipped with holes for the passage of fixation screws. At least in the areas defining the screw holes, the aforementioned plate is made from a material having mechanical properties such that the periphery of the holes can be self-tapped by means of tapping screws which can be used to fix the plate.

The present invention provides an advance in the design of an assembly which allows a fastener or fixator, including for example, a screw, or peg, to be inserted through a threaded hole in a stabilizer or implant, such as a plate, anchor or cage, at a variable angle in order to best capture a bone or bone segment with the fastener. The angle of the fastener can subsequently be locked to fix the bone or bone segment relative to the plate, or to fix the plate relative to the bone or bone segment. The invention allows for at least about 10°, and more preferably 15°, of angulation relative to a longitudinal axis of the opening that the fastener is inserted through. The assembly provides the advantage of providing the option to the surgeon of using a locking screw having a threaded head which mates with the threads in the hole to fix the screw at a pre-determined angle, to use a non-locking, non-threaded hemispherical headed screw which is sized and shaped to ride on the threads to hold the plate in compression relative to the bone when the screw is implanted at a desired variable angle or to use the assembly which comprises a threaded bushing in the hole with a threaded screw at a variable angle relative to the hole.

There are numerous implant applications that can benefit from a variable angle locking mechanism. Specific examples include use in the small bones, i.e. those bones distal to the elbow and knee and the clavicles, although it is understood, that the mechanism can also be of use in other areas of the body, including the long bones, the pelvis and the spine.

In addition, the orthopedic implant system of the present disclosure includes a fastener driver in particular for use with a variable locking assembly in which the driver has a variable angle drive tip and a depth limiting sleeve that helps to inhibit the user from driving the fastener too deeply within the plate hole.

### SUMMARY OF THE INVENTION

According to the invention, an orthopedic plate system according to claim 1 is provided. Preferred embodiments of the invention are set forth in the dependent claims. The orthopedic system of the present invention includes an implant, and preferably a plate having a threaded fastener hole and three types of fasteners that can interchangeably be used in the threaded fastener hole, specifically, fixed angle locking fastener having a threaded head that mates with the threads of the fastener hole to lock the fastener into the plate at a fixed angle, a variable angle non-locking fastener that has a rounded head that rides on the threads of the fastener hole at a variable angle and holds the plate relative to the bone into which the fastener is implanted by compression between the fastener head and the plate hole, and an assembly that provides for variable angle fastener locking. Advantageously, the non-locking fasteners have convexly rounded heads that are sized to fit within the threaded hole at a variable angle providing for about 20° of conical rotation relative to the longitudinal axis of the fastener hole. The variable angle assembly includes a locking insert that is threaded into, and thus mechanically seated in, the internally threaded opening in the plate. The locking insert has an annular flange or shoulder that surrounds the opening in the plate on the superior surface (relative to the associated bone or bone fragment), and also has a central through opening that is preferably smooth and hexagonal in cross section to allow the insert to be screwed into the plate opening. The locking insert is made from a biocompatible material that can be deformed by the threads of the fastener head, (i.e. a screw or peg,) so that the fastener is inserted through the locking insert and into the adjacent bone to the point where the proximal head threads interact with the internal opening of the locking insert to cause the material to flow and accept the head of the fastener at any angle (up to ∼15°) while maintaining a rigid construct. Both the minor diameter and the major diameter of the fastener head include a taper to improve the locking within the locking insert. Preferably, the major diameter of the threads taper out at a larger angle than the minor diameter, i.e. by a difference of from about 2° to about 20°, preferably from about 5° to about 15°, and preferably about 8° to about 12° degrees of difference, so that the threads widen at a greater rate than the head does to improve the feeling when the fastener is locked into the insert.

Thus, the disclosure relates to a surgical tray or caddy that includes orthopedic plates which have internally threaded fastener holes, a variable locking insert which threads into the threaded fastener hole and which has a through opening and an externally threaded fastener that can be inserted into the locking insert to deform the surface of the through opening to lock the fastener at a selected angle relative to the fastener hole, and at least one of a fastener having a head with threads that are capable of mating with the threads of the fastener hole or a fastener that has a convexly rounded surface that is sized to fit with the fastener hole and hold the plate but also to allow for a variable angle position of the fastener within the fastener hole. The disclosure relates to a fastener driver which has a drive mechanism which includes a depth limiting feature that has an interference type drive clutch with a spring that biases the depth limiting assembly into engagement and that disengages when the fastener is inserted to a desired depth (in particular when the terminus of a depth limiting sleeve (joined by threads to a drive couple member) encounters a surface surrounding the fastener aperture which counters the bias of the spring to disengage the clutch assembly.

The fastener driver also includes a replaceable drive tip having a detent, which is preferably a coil spring that snaps into an annular groove within a drive feature housed within the depth limiting sleeve. The drive tip unit also includes a drive tip that floats within the driver tip unit at a variable angle to allow a fastener to be driver at an angle relative to the long axis of the drive train of the fastener driver. Specifically, the drive tip comprises a rounded end, which is advantageously a drive ball (i.e. a hex ball) or curving portion joined at ridges such that the cross-section of the rounded end of the tip in a plane transverse to the long axis forms a hex (although other geometries such as a pentagon or square or even a hexalobe could be used). The drive shaft of the drive tip unit includes a corresponding recess that has a shape including angles that are the inverse of the ridges of the drive ball that capture the drive ball to transmit torque to the drive end of the drive tip, which can be any appropriate geometry to correspond to the torque receiving recess of the fastener. In this case, the drive end is shown as a hexagon, but advantageously drive tip units can be supplied within a given system or surgical tray to accommodate various torque driving recesses, includes various sized hexagons, hexalobes, or even Phillips head fasteners.

In addition, a fastener system is disclosed for use in orthopedic surgery which includes an orthopedic implant, such as an orthopedic plate having apertures that receives fasteners, and specifically includes variable locking apertures, in combination with a fastener driver that has a depth limiting feature, such as a clutch that disengages to inhibit the user from driving the fastener or fastener assembly too deep relative to the implant. Similarly, a method is disclosed of promoting the achievement of variable angle fixation within an orthopedic plate comprising providing in an orthopedic tray, an orthopedic plate having a fastener aperture with an aperture axis, a fastener having a longitudinal axis which is capable of being implanted within said fastener aperture with its longitudinal axis at a variable angle relative to the aperture axis, and a fastener driver having a drive axis and which includes a mechanism that disengages to inhibit the fastener to be driven too deep relative to the plate, and optionally the fastener driver includes a variable angle drive tip that allows the tip to be used at a variable angle relative to the "drive axis" of the fastener driver (which is used herein to mean the long axis of the driver that defines the rotational axis through which the driver is driven).

The present disclosure achieves the foregoing goals within the fastener driver by providing a drive train comprising a drive shaft which is coupled to a handle for manual use or to a motor in the event that a power drive is desirable, and which has a housing for the shaft of a drive feature or linking section with a rectilinear (preferably having from 3 to 10 sides, and more preferably 4 to 8 or 6) boss section about an opening that houses the drive train, that forms an interference fit in a corresponding recess at the forward end of the drive couple. The drive feature further includes a second housing having a composite recess that accepts the end of a drive tip unit for easy replacement. A spring, which is preferably a coil spring, but could also include other biasing means, is biased between the front section of the first housing and at the other end against the rear end of a recess within a drive couple that has a front annulus including external threads that mate with the internal threads of a depth limiting sleeve which surrounds the second housing, as well as the drive tip unit up to the drive portion of the drive tip of the drive tip unit. The projecting drive portion of the drive tip unit engages the torque receiving recess of the fastener, and allows the fastener driver to be used to drive the fastener into an orthopedic plate. The sleeve has a terminal annular flat nose surrounding the drive tip, which will cause the drive train to uncouple by counteracting the spring to disengage the interference fit between the drive couple and the drive feature, essentially disengaging the clutch mechanism of the fastener driver of the present invention.

### BRIEF DESCRIPTION OF THE DRAWINGS

Figure 1 is an exploded view of a variable locking assembly in accordance with the present invention with the plate illustrated in section;
Figure 2 is a view from the top and the side of the variable locking assembly of the present invention;
Figure 3 is a side view of the screw head of Figure 1;
Figure 4 is a top side view of variable locking insert of figure 1;
Figure 5 is a cross section of the variable locking assembly insert of Figure 2 taken along line 5-5;
Figure 6 is a cross section of the plate shown in Figure 1 with a threaded locking screw;
Figure 7 is a top side view of a bone plate and fastener system illustrating the use of all three modes of fixation provided by the present invention;
Figure 8 is a cross-section of the bone plate of Figure 7 taken along line 8-8;
Figure 9 is view of the variable non-locking fixation of the present invention taken from the same cross-sectional view as for Figure 8;
Figure 10 is a top side view of the locking insert driver of the present disclosure;
Figure 11 is a distal end view of the locking insert on the insertion tool for the variable locking assembly of the present invention;
Figure 12 is a cross-section distal end view of the locking insert on the insertion tool for the variable locking assembly of the present invention;
Figure 13 is top side view of the variable angle non-locking screw of the present invention;
Figure 14 is top side view of the fixed angle locking screw of the present invention;
Figure 15 is top side view of the variable angle locking screw assembly of the present invention;
Figure 16 is a side view of the fastener driver of the present disclosure;
Figure 17 is a cross section of the fastener driver shown in Figure 16 taken along line 16-16;
Figure 18 is an exploded side view of the fastener driver of Figure 16;
Figure 19 is a first side view of a drive tip for use in the present and
Figure 20 is an end view of the drive tip of Figure 19 taken at a 90° rotation.

### DETAILED DESCRIPTION OF THE DRAWINGS

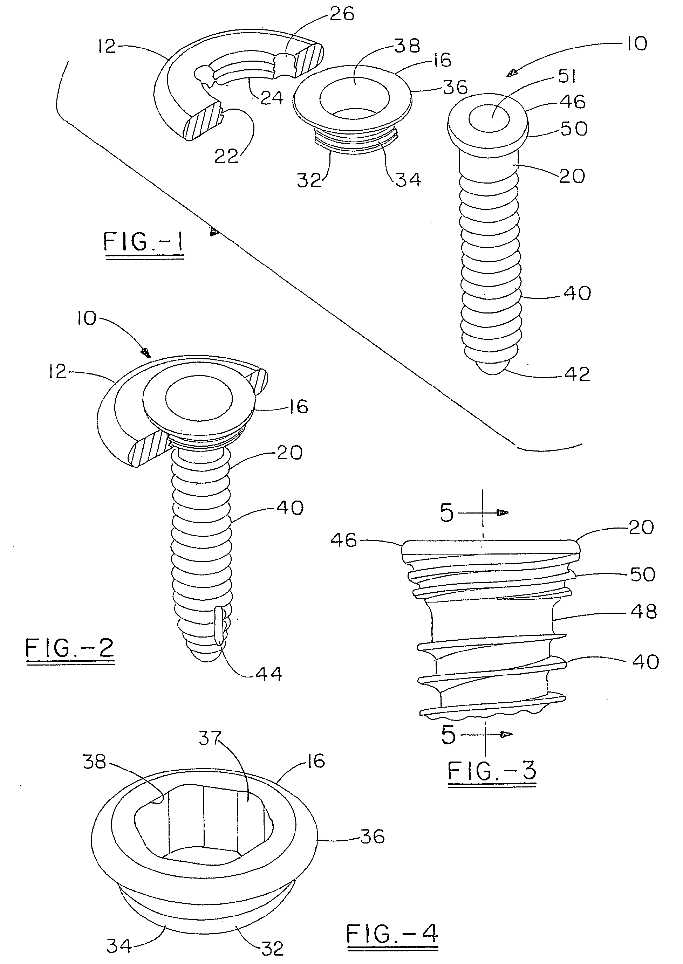

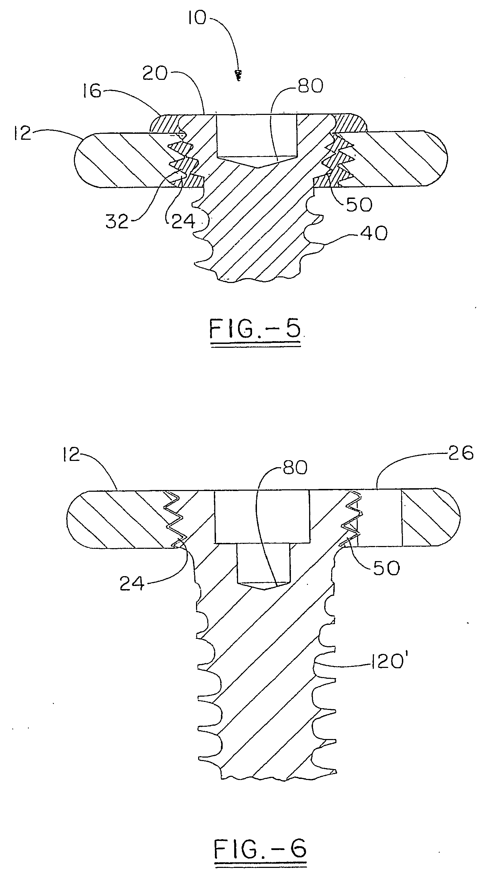

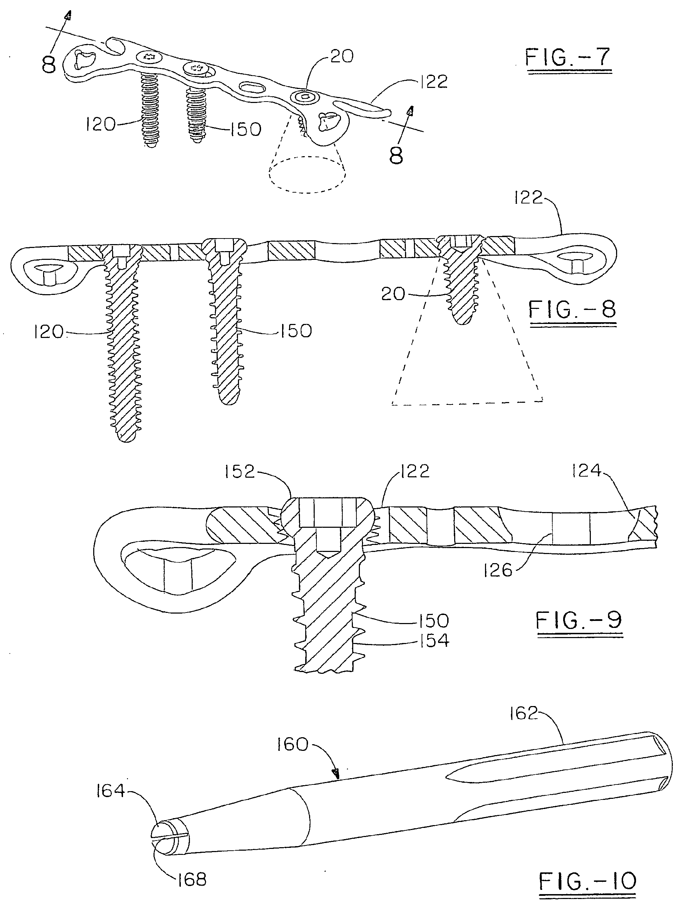

Figure 1 shows an exploded view of a variable axis locking mechanism assembly in accordance with the present invention which are further illustrated in Figures 2 through 6. Figure 5 shows a cross section of the assembly of Figure 1. The assembly 10 includes an implant member which is shown as a plate 12, a locking insert 16 and a variable locking fixator, which is shown as a screw 20. For the purpose of this description, the plate is shown as part of an annular portion of a plate including a through hole 22 with internal threads 24, and optionally grooves 26 that act as seats for a drill guide having mating cylindrical projections.. The plate could have any appropriate shape according to the application with which it is used, distal radius plates, calcaneal plates, long bone plates, plates for the clavicles, spinal plates, plates for use in the hand and foot, or any other surgical implant and accordingly is illustrated in Figures 7-9 as having a modified X-shape where the plate has diagonally opposed pairs of long and short arms that each include a threaded fastener hole. The plate is further shown as including one or more compression holes in the middle of the plate.

The plate generally has a top side, or superior side, i.e. the side which faces away from the bone in use, with a generally constant through thickness to a bottom side, or side that faces toward the bone in use. The plate can be planar, or have another topography, according to the application, although the through hole portion 22 must have a topography that allows the through hole of being capable of receiving the locking insert 16 and the threads 24 of the hole are capable of mating with the external threads 32 of the body 34 of the locking insert or the head of a fastener designed for fixed angle locking. As illustrated in Figure 4, the locking insert includes an annular flange member 36, which generally forms a shoulder area that seats against the top portion of the plate that surrounds the through hole 22. The locking insert further includes an internal opening 38 that is advantageously smooth and which has a cross-sectional configuration that allows the locking insert to be screwed into the plate hole. Preferably, this is a hexagonal shape having internal edges where the flats join that are smoothed out to better accommodate the screw head. The locking insert is made from a biocompatible material such as nylon or PEEK polymer (i.e., polyether ether ketone) or other suitable biocompatible polymer, that is softer than the screw so that when the screw head is screwed into the locking insert, the external threads will cause the surface of the internal opening 38 to flow or deform to form threads in the locking insert, causing the screw to lock into position relative to the plate in the locking insert. Also advantageously, the locking insert 16 is provided in the surgical tray pre-mounted on a locking insert driver tool 160 shown in Figures 10-12. The tool 160 is similar in shape to the drill guide that is used with the system, and comprises a long hollow tube having a cannula which can receive and guide a guide for the drill used for the fastener that is used with the locking insert. The outside of the distal end of the tube fits into the through hole 22 of the locking insert 16 and is shaped such as in a hex shape corresponding to the shape of the internal opening 38 of the insert and tapered so as to be sufficiently secure to permit the tool to be used to drive the insert 16 into the plate 12.

Figures 1, 2 and 3 show a variable axis locking fastener which is a screw 20 but which could also be a variable axis locking peg. The screw includes a portion having a thread 40 for attachment within a bone or bone segment. The screw can include an insertion tip 42 that has a point, or as shown, a blunt tip with optional cutting flutes 44. The screw has a head portion 46 that is joined to the distal threaded portion by an area 48 having threads of a smaller major diameter and also including an area that is free from threads or is cylindrical. The head of the screw includes external threads 50 where the minor diameter and major diameter both taper, but preferably, the major diameter tapers at a smaller angle than the minor diameter so that the threads become thicker as they progress toward the top end of the screw. The minor diameter tapers at an angle of from about 20° to about 60°, and preferably from about 30° to about 50° degrees and most preferably about 35° to about 45° while the major diameter tapers at an angle of from about 30° to about 70°, and preferably from about 40° to about 60° degrees and most preferably about 45° to about 55° with an advantageous differential being about 8° to about 12°. The head portion 46 further includes a torque driving recess 52, with an optional bore 80 which retains the screw 54 on the post of a screwdriver.

Figure 6 illustrates the plate of the present invention accepting a locking screw which has threads on the exterior surface of the head that mate with the internal screws of the through hole in the plate.

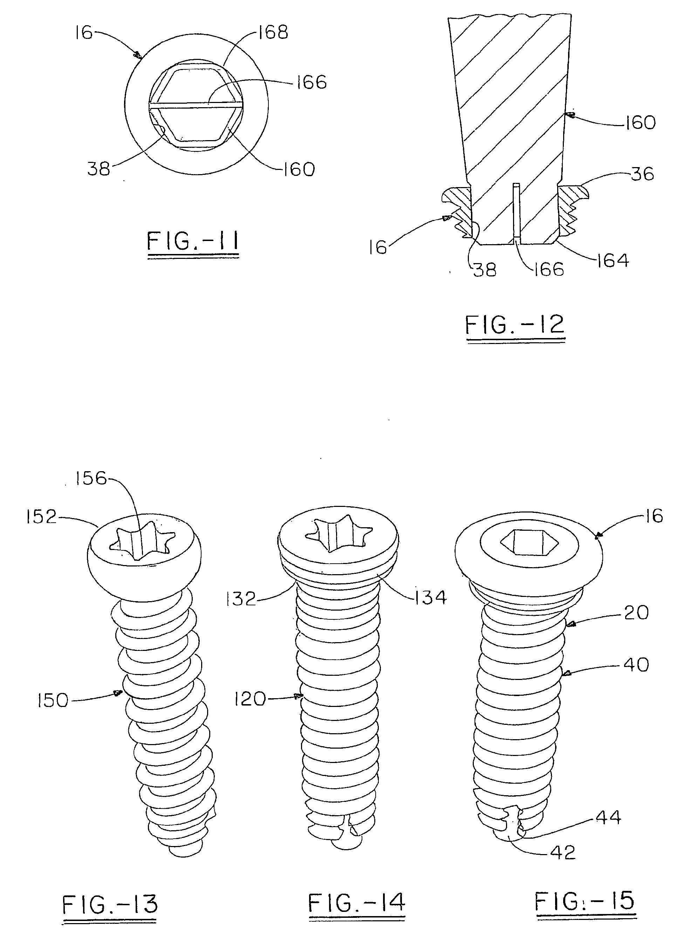

Figures 7-9 illustrate a modified X-shaped plate 122 that has threaded locking screw holes 122 and compression holes 124 that include a shoulder 126 to cause compression of the plate as the screw is driven down into the plate hole. This plate 12' is illustrated as including three modes of fixation, using a variable locking screw assembly 20, a threaded locking screw 120 and a non-locking variable screw 150. These three types of screws are better illustrated in Figures 13-15. Figure 13 shows the non-locking screw 150 which has a rounded head 152 that rides on the internal threads of the fastener hole 22 within the plate as is shown in Figure 9 in detail. Once again, the screw 150 further includes a torque driving recess 156 and bone threads which have a lower pitch then the other two types of screws provided with the present invention in order to provide better purchase in the bone. The screw has a head portion 152 that is convexly rounded, and preferably hemi-spherical and sized so as to ride on the internal threads of the plate and to secure the plate to the bone, but to allow an conical range of fixation of about 10° to about 20 °, and preferably about 12° to about 17°, and more preferably 15°, (which is lower than the angle permitted by the locking variable angle assembly since a higher angle contributes to lower strength. In order to compensate for the lower strength, the non-locking variable locking screw has a bone screw portion154 that has a lower pitch and a slightly greater major diameter, with the same size minor diameter.

Figures 10 - 12 illustrate a locking insert driver 160 that can be used to install the insert in the plate of the present invention. The driver has a handle 162 and a tip 164 that includes a split 166 and ridges 168 which fit into radiused areas 37 within the locking ring insert through hole 38 in order to drive the insert into the through hole of the plate. The split allows the driver tip to compress to form a tighter friction fit of the insert on the driver.

The present invention is novel in providing a surgical caddy that includes a plate with threaded hole and a variable locking assembly that has a threaded deformable locking insert and a fastener having a threaded head that can be inserted in the locking insert to lock it into position, and either or both a locking fastener that has a threaded head that locks into the threaded hole to lock the fastener at a fixed angle, or a variable locking fastener that has a convexly rounded head that is sized to fit within the threaded hole so that the non-locking fastener can be inserted at a variable angle, but which secures the plate to the bone, but is not mechanically coupled to the plate. Thus, the surgeon is provided with a single plate, basically a single surgical procedure with very little change to provide selectable fixation by choosing the fastener. Testing was performed on the variable locking mechanism of the present invention showing cyclic loading of the variable locking assembly of the present invention at an applied load of 35, 55, and 67.5 Newtons at a run cycle to failure or one million cycles. Only one of the assemblies of the present invention failed below the test end, i.e. at more than 300,000 cycles, while the majority of the comparative product failed at an average of 22,000 with loading at 55 N. The test results are shown below in Table 1.

**Table 1**

| **Customer Spec ID** | **% Static Peak Load** | **Applied Load** | **Applied Moment** | **Cycles Tested** | **Dynamic Stiffness** | **Observations and/or Failure mode** |
|---|---|---|---|---|---|---|
| | | **(N)** | **(N-m)** | **(n)** | **(N/mm)** | |
| **Stryker 15°** | Cust P | 67.50 | 0.7425 | 71,102 | 22.92 | a |
| **Stryker 15°** | Cust P | 35.00 | 0.385 | 1,000,000 | 78.75 | b |
| **Stryker 15°** | Cust P | 55.00 | 0.605 | 30,530 | 70.71 | a |
| **Stryker 15°** | Cust P | 55.00 | 0.605 | 29,596 | 78.57 | a |
| **Stryker 15°** | Cust P | 55.00 | 0.605 | 44,539 | 99.00 | c |
| **Stryker 0°** | Cust P | 55.00 | 0.605 | 7,790 | 47.60 | c |
| **Stryker 0°** | Cust P | 55.00 | 0.605 | 15,472 | 58.24 | c |
| **Stryker 0°** | Cust P | 55.00 | 0.605 | 3,291 | 55.62 | c |
| **Ortho 15°** | Cust P | 55.00 | 0.605 | 1,000,000 | 88.39 | b |
| **Ortho 15°** | Cust P | 55.00 | 0.605 | 1,000,000 | 86.84 | b |
| **Ortho 15°** | Cust P | 55.00 | 0.605 | 317,842 | 88.39 | d |
| **Ortho 0°** | Cust P | 55.00 | 0.605 | 1,000,000 | 93.40 | b |
| **Ortho 0°** | Cust P | 55.00 | 0.605 | 1,000,000 | 90.00 | b |
| **Ortho 0°** | Cust P | 55.00 | 0.605 | 1,000,000 | 85.34 | b |

| | | | | | | |
|---|---|---|---|---|---|---|
| a 2 mm of displacement, screw head pulled through plate, plate fracture b no observed failure c partial fracture of plate d partial separation of peek ring from plate | | | | | | |

The fastener driver of the present disclosure is preferably intended for use in surgically implanting a bone fastener to a bone to retain the bone or bone fragment relative to an orthopedic implant, which could include various constructs such as cages, rods, and plates, but which is envisioned primarily for use with orthopedic plates. Similarly, the fastener with which the driver is intended for use could include various types of fasteners, such as screws and pegs (i.e. with and without bone threads), but for the sake of expediency, when the term "screw" is used herein, it should be interpreted to include fasteners without terminal threads, such as are sometimes referred to as pegs.

Further, the driver of the present disclosure is used advantageously with a variable angle or polyaxial fastener system in which the fastener, i.e. screw, is inserted into an aperture within the implant, i.e. plate, with its long axis at a variable angle relative to the central axis of the aperture. The present invention encompasses a plate/fastener system and method of enabling surgery which involves a plate having a threaded fastener aperture that accepts a variety of fasteners including a locking fastener having a threaded head which mates with the threads of the fastener aperture, or a variable angle non-locking fastener which has a partially spherical head that rides on the internal threads of the fastener aperture, or a variable angle locking assembly that includes a threaded locking insert that mates with the threaded fastener aperture and which comprises a material that will flow or deform relative to the threads of the head of a variable angle locking fastener that is threaded into the locking insert. Additionally, the present disclosure includes a fastener driver that has a depth limiting drive mechanism that will inhibit the user from driving any of these fasteners through the plate or too deep within the fastener aperture. The disclosure also relates to a method of enabling surgery comprising providing an orthopedic plate system that includes an orthopedic plate having at least one threaded fastener aperture, a fastener that can be threaded into the fastener aperture, and a fastener driver that includes a clutch that will disengage if the fastener is driven below a desired depth relative to the plate aperture. Where the plate allows for variable locking fixation using a locking insert as previously described, it should be understood that the locking insert could be considered to be part of the plate so as to define the fastener insert, or could be considered to be part of the fastener.

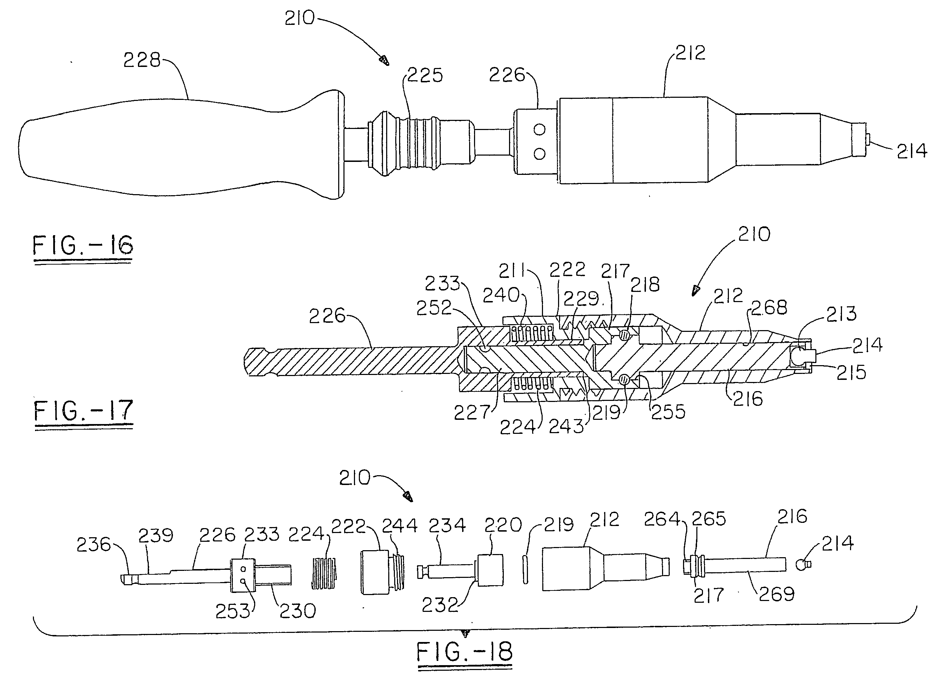

Figure 16 illustrates a side view of the fastener driver of the present disclosure, which is further shown in cross-section is Figure 17 and as an exploded view in Figure 18. The driver 210 includes a disengageable drive mechanism 211 which includes a sleeve 212 that houses a replaceable drive tip unit 213. The replaceable drive tip unit 213 includes a drive tip 214, which is held in a recess 215 within a drive shaft 216. The drive shaft 216 is crimped around the rounded portion of the drive tip 214 so as to allow the drive tip to rotate in the recess 215, but to retain it axially from dislodging from the drive shaft 216. The drive shaft 216 has a shaft portion joined to a drive body 217, which includes a groove 218 that receives a detent spring 219.

The drive shaft 216 is removably inserted into and mechanically coupled (by means of an interference fit) to a compound recess within a drive feature or link 220 which is pinned within a drive connector 226. A drive couple 222 houses and mechanically couples the drive feature 220 with the drive connector 226 that is directly manually driven by means of the handle2 28 shown as including an optional ratcheting mechanism 225 . The drive connector 226 further includes a square shaft 230 which has the cylindrical recess 227 that receives the shaft portion2 29 of the drive feature 220, and which is captured within the square recess 231 of the drive couple 222. The square recess 231 of the drive couple also accepts a square boss 232 on the rear face of the housing portion of the drive feature 220. It is this coupling between the square recess 231 of the drive couple 222 and the square boss of the drive feature that transmits the drive torque to the drive tip unit 213, and ultimately drives the fastener.

The drive connector 230 also includes an annulus 233, which includes the terminus of the cylindrical recess 227 that accepts the cylindrical shaft 234 of the drive feature 220. Specifically, the shaft includes hollow section 251 that accommodate pins 252 held in pin openings 253 within the annulus 233 of the drive connector 226. This connection links the drive feature 220 both axially and radially to the drive connector 226.

A coil spring 224 is biased at one end against a front shoulder of the annulus 233 of the drive feature 226, and at the other end at the rear shoulder formed within a larger diameter recess 240 within the drive couple 222. The coil spring biases the drive couple 222 and the sleeve 212, which has a threaded connection to the drive couple forward relative to the drive feature (or in the direction toward the drive tip 214). The drive couple 222 has a front nipple 244 that includes external threads 246 that mate with the internal threads 250 within the bore of the sleeve 212.

The drive shaft 226 also includes a terminal shaft portion 236, which is adapted to be received within the recess 238 of the drive handle 228. One typical connection includes a flat 239 that permits the transmission of torque from the handle to the drive mechanism.

The axial play 243 between the square shaft 230 of the drive connector 230 and the correspondingly sized square boss 232 on the rear face of the drive feature within the square recess of the drive couple is what defines the disengagement of the drive mechanism, and forms the essence of the interference clutch mechanism of the fastener driver of the present disclosure.

The drive feature 220 has a compound recess 255 that includes an annular groove 256, which accepts a detent spring 218 to hold the replaceable drive tip unit within the fastener driver. The rear end 257 of the recess 255 is square and accepts a corresponding square drive shape 264 at the rear end of the drive shaft 216 of replaceable drive unit 213. Forward of the square section is a drive shaft body 265 which includes an annular groove 218 to also accommodates the detent spring 218 which is held within the groove 256 in the wider diameter portion of the compound recess within the drive feature.

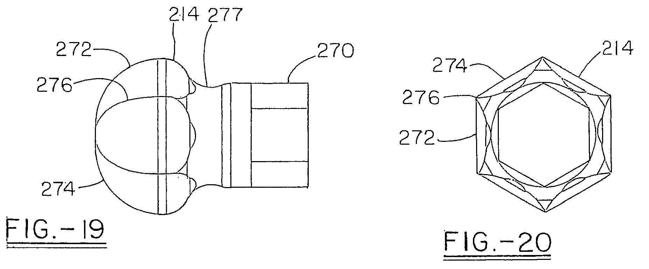

The sleeve 212 includes an internal recess 268 that houses the drive feature 220 and the shaft 269 of the drive tip unit 213 and ends in an annular flat surface or nose 275. The shaft 269 of the drive tip unit includes a recess 215 that is hexagonal in cross-section so as to accept and transmit torque to the hex-ball section 272 of the drive tip 214. As is shown in Figures 19 and 20, the drive tip includes a section that includes rounded surface 274 that join at ridges 276 to permit the drive tip to be driven within the recess 215 of the drive tip unit and a necked portion 277 that accommodates the terminus of the recess 215. Thus, the drive tip can be driven at a variable angle of conical rotation relative to the drive axis of the fastener driver. The variable angle of conical rotation is from about 2° to about 30°, and preferably from about 5° to about 20°, and most preferably from about 7° to about 15°, since the tip is free to float on the rounded surfaces within the recess. The end 270 of the drive tip is a typical drive shape, and while it is illustrated as hexagonal, can encompass other drive shapes, including for example, hexalobe, star shape, square, and cross-shaped.

While in accordance with the patent statutes the best mode and preferred embodiment have been set forth, the scope of the invention is not limited thereto, but rather by the scope of the attached claims.

## Claims

1. An orthopedic plate system comprising:
an orthopedic plate (12) including an longitudinal axis and a first through hole (22) defining a plate internal wall with threads (24) and an external surface, a fixed angle locking fastener and a variable angle locking fastener, **characterized in that** the orthopedic plate system further comprises:
a variable angle locking mechanism assembly comprising a deformable locking insert (16) having an insert internal wall defining an internal second through hole (38) having a cross-sectional configuration that includes flat surfaces and forming a torque driving recess and the locking insert having external threads (32) that mate with the threads (24) of the first through hole of the plate, and the variable angle locking fastener (20) having a portion for attachment in bone, and a head portion having external threads (50), the insert internal wall comprising a material that will deform relative to the material of the external threads of the head portion of the fastener so that the fastener head will seat in the locking insert in a locked relation to the plate; wherein the first through hole in the plate can optionally accommodate the fixed angle locking fastener or the locking insert (16).

2. The orthopedic plate system as set forth claim 1 wherein the insert (16) further includes an annular flange (36) about the through hole which forms a shoulder that mates against an external surface of the plate.

3. The orthopedic plate system as set forth in claim 1, wherein the locking insert (16) is made of peek and the fastener (50) is made of a metal.

4. The orthopedic plate system assembly as set forth in claim 1, wherein the through hole (38) in the locking insert (16) has a cross-section that forms a polygon having from 4 to 10 sides.

5. The orthopedic plate system as set forth in claim 1, wherein the threaded portion (50) of the head has a minor diameter which has a taper and a major diameter which has a taper and wherein the minor diameter taper is larger than the taper of the major diameter.

6. The orthopedic plate system as set forth in claim 5, wherein the difference in the taper of the major diameter to the minor diameter is from about 3° to about 20°.

7. The orthopedic plate system as set forth in claim 1 further comprising a variable non-locking fastener which has a rounded head portion and the head portion does not include external threads.

## Patentansprüche

1. Orthopädisches Plattensystem, aufweisend:
eine orthopädische Platte (12), aufweisend eine Längsachse und ein erstes Durchgangsloch (22), das eine Platteninnenwand mit Gewinde (24) und eine Außenfläche definiert, ein Fester-Winkel-Sperr-Befestigungselement und ein Variabler-Winkel-Sperr-Befestigungselement, **gekennzeichnet dadurch, dass** das orthopädische Plattensystem weiter aufweist:
eine Variabler-Winkel-Sperrmechanismus-Vorrichtung, aufweisend einen deformierbaren Sperreinsatz (16), der eine Einsatzinnenwand aufweist, die ein inneres, zweites Durchgangsloch (38) definiert, welches eine Querschnittskonfiguration hat, die glatte Flächen aufweist, und eine Drehmoment-Antriebs-Vertiefung ausbildet, wobei der Sperreinsatz ein Außengewinde (32) aufweist, das mit dem Gewinde (24) des ersten Durchgangslochs der Platte zusammenpasst, wobei das Variabler-Winkel-Sperr-Befestigungselement (20) einen Abschnitt zur Befestigung in Knochen aufweist, und wobei ein Kopfabschnitt ein Außengewinde (50) aufweist, wobei die Einsatzinnenwand ein Material aufweist, das sich bezogen auf das Material des Außengewindes des Kopfabschnitts des Befestigungselements verformen wird, so dass der Befestigungselementkopf in dem Sperreinsatz in einer gesperrten Relation zu der Platte sitzen wird, wobei das erste Durchgangsloch in der Platte wahlweise das Fester-Winkel-Sperr-Befestigungselement oder den Sperreinsatz (16) aufnehmen kann.

2. Orthopädisches Plattensystem gemäß Anspruch 1, wobei der Einsatz (16) weiter um das Durchgangsloch herum einen ringförmigen Flansch (36) aufweist, welcher eine Schulter ausbildet, die gegen eine Außenfläche der Platte passt.

3. Orthopädisches Plattensystem gemäß Anspruch 1, wobei der Sperreinsatz (16) aus PEEK hergestellt ist und das Befestigungselement (50) aus einem Metall hergestellt ist.

4. Orthopädisches-Plattensystem-Vorrichtung gemäß Anspruch 1, wobei das Durchgangsloch (38) in dem Sperreinsatz (16) einen Querschnitt aufweist, der ein Polygon ausbildet, welches von 4 bis 10 Seiten aufweist.

5. Orthopädisches Plattensystem gemäß Anspruch 1, wobei der Gewindeabschnitt (50) des Kopfes einen kleinen Durchmesser, welcher eine Verjüngung aufweist, und einen großen Durchmesser hat, welcher eine Verjüngung aufweist, und wobei die Verjüngung des kleinen Durchmessers größer ist als die Verjüngung des großen Durchmessers.

6. Orthopädisches Plattensystem gemäß Anspruch 5, wobei die Verjüngungs-Differenz vom großen Durchmesser zum kleinen Durchmesser von etwa 3° bis etwa 20° beträgt.

7. Orthopädisches Plattensystem gemäß Anspruch 1, weiter aufweisend ein variables nicht sperrendes Befestigungselement, welches einen abgerundeten Kopfabschnitt aufweist, wobei der Kopfabschnitt keine Außengewinde aufweist.

## Revendications

1. Système de plaque orthopédique comprenant :
une plaque orthopédique (12) comprenant un axe longitudinal et un premier trou débouchant (22) définissant une paroi interne de plaque avec des filetages (24) et une surface externe, une fixation de blocage à angle fixe et une fixation de blocage à angle variable, **caractérisé en ce que** le système de plaque orthopédique comprend en outre :
un ensemble de mécanisme de blocage à angle variable comprenant un insert de blocage déformable (16) ayant une paroi interne d'insert définissant un second trou débouchant interne (38) ayant une configuration transversale qui comprend des surfaces plates et formant un évidement d'entraînement de couple et l'insert de blocage ayant des filetages externes (32) qui se couplent avec les filetages (24) du premier trou débouchant de la plaque, et la fixation de blocage à angle variable (20) ayant une partie pour la fixation dans l'os, et une partie de tête ayant des filetages externes (50), la paroi interne d'insert comprenant une matériau qui se déforme par rapport au matériau des filetages externes de la partie de tête de la fixation de sorte que la tête de la fixation s'installe dans l'insert de blocage dans une relation bloquée par rapport à la plaque ; dans lequel le premier trou débouchant dans la plaque peut facultativement loger la fixation de blocage à angle fixe ou l'insert de blocage (16).

2. Système de plaque orthopédique selon la revendication 1, dans lequel l'insert (16) comprend en outre une bride annulaire (36) autour du trou débouchant qui forme un épaulement qui se couple contre une surface externe de la plaque.

3. Système de plaque orthopédique selon la revendication 1, dans lequel l'insert de blocage (16) est réalisé à partir de polyétheréthercétone et la fixation (50) est réalisée à partir d'un métal.

4. Ensemble de système de plaque orthopédique selon la revendication 1, dans lequel le trou débouchant (38) dans l'insert de blocage (16) a une section transversale qui forme un polygone ayant de 4 à 10 côtés.

5. Système de plaque orthopédique selon la revendication 1, dans lequel la partie filetée (50) de la tête a un diamètre mineur qui a une conicité et un diamètre majeur qui a une conicité, et dans lequel la conicité du diamètre mineur est supérieure à la conicité du diamètre majeur.

6. Système de plaque orthopédique selon la revendication 5, dans lequel la différence de conicité du diamètre majeur par rapport au diamètre mineur est d'environ 3° à environ 20°

7. Système de plaque orthopédique selon la revendication 1, comprenant en outre une fixation sans blocage variable qui a une partie de tête arrondie et la partie de tête ne comprend pas de filetages externes.
